# EUROPÄISCHE PATENTSCHRIFT

(11) **EP 2 339 934 B1**
(45) Veröffentlichungstag und Bekanntmachung des Hinweises auf die Patenterteilung: **29.08.2018**
(21) Anmeldenummer: 09752733.7
(22) Anmeldetag: 26.10.2009
(51) Int. Cl.: A41B 11/02

(54) **KOMPRESSIONSKLEIDUNG**
COMPRESSION CLOTHING
VÊTEMENT DE COMPRESSION

(30) Priorität: 24.10.2008 DE 202008014202 U
(43) Veröffentlichungstag der Anmeldung: 06.07.2011
(73) Patentinhaber: X-Technology Swiss GmbH, 8832 Wollerau (CH)
(72) Erfinder: LAMBERTZ, Bodo, W., CH-8808 Pfäffikon (CH)
(74) Vertreter: Schneider Feldmann AG Patent- und Markenanwälte
(86) Internationale Anmeldenummer: PCT/EP2009/007633
(87) Internationale Veröffentlichungsnummer: WO 2010/046130

(56) Entgegenhaltungen:
- EP-A1- 1 475 062
- WO-A1-2010/025186
- WO-A2-2005/094738
- FR-A1- 2 852 509
- JP-A- 2006 225 800
- JP-A- 2007 063 722
- Nicola Oberlinger: "translation japanese to german of JP2006225800" In: "translation japanese to german of JP2006225800", 13 February 2014 (2014-02-13), XP055384820, vol. 3
- Ingeborg Von Der Grim Kitaoka: "translation japanese to german of JP2007063722" In: "translation japanese to german of JP2007063722", 28 March 2015 (2015-03-28), XP055384822, vol. 3

## Beschreibung

Die Erfindung betrifft einen als Kompressionskleidungsstück ausgebildeten Kniestrumpf mit den Merkmalen des Oberbegriffs des Anspruchs 1

Kompressionskleidung erzeugt von außen Druck auf den Körper; bei Kompressionsstrümpfen wird beispielsweise ein Druck auf das Gewebe des umschlossenen Beines erzeugt. Kompressionsstrümpfe sind dabei so gefertigt, dass der ausgeübte Druck von oben nach unten analog zum Gewebedruck in Richtung der Schwerkraft zunimmt. Der ausgeübte Druck der Kompressionskleidung ist je nach Art der Behandlung wählbar. Der von der Kleidung ausgeübte Druck ist in Kompressionsklassen eingeteilt.

Die Verwendung von Kompressionskleidung ist einteilbar in medizinische und kosmetische bzw. prophylaktische Anwendungen. Mit der medizinischen Anwendung werden beispielsweise Krampfadern (Varizen), Beinvenenthrombosen und dergleichen behandelt. Mit prophylaktischen Anwendungen, bei denen die Kompressionskleidung lediglich einen geringen Druck auf den Körper ausübt, werden beispielsweise "Reisethrombosen" behandelt oder sie werden als Unterstützung für Angehörige dauernd stehender Berufe genutzt. Durch die prophylaktische Anwendung kann das Risiko einer Thrombose beispielsweise auf Langstreckenflügen wesentlich reduziert werden. Zudem findet Kompressionskleidung, insbesondere Kompressionsstrümpfe, im Sport Anwendung, beispielsweise beim Nordic-Walking oder Marathonlaufen.

Die bekannte Kompressionskleidung übt einen flächigen Druck auf den Körper aus. Dadurch ist zwar eine gleichmäßige Druckverteilung hervorgerufen; es ist jedoch gleichzeitig eine Einschnürung der Blutversorgung hervorgerufen, die zwar bei medizinischen Anwendungen wünschenswert sein kann; bei prophylaktischen Anwendungen, insbesondere im Sport, ist dies jedoch aus folgendem Grund unerwünscht:
Als Folge muskulärer Arbeit steigt bei (Ausdauer-)Belastungen die Muskeltemperatur deutlich an. Mit dem Blutstrom wird die produzierte Wärme aus dem Muskel in den Organismus transportiert, wodurch die Körperkerntemperatur ebenfalls ansteigt. Durch die Erwärmung erweitern sich die Kapillare unter der Haut. Dies ist zum Beispiel Ursache für Hautrötungen bei starken körperlichen Anstrengungen, beispielsweise das Erröten des Gesichts. Zur Reduzierung der Bluttemperatur findet im Körper eine Blutumverteilung statt. Das sauerstoffreiche Blut wird dabei aus dem Körperzentrum in die Peripherie, also in Richtung der Haut, verlagert, wo eine Abkühlung erfolgt. Das Blut fungiert quasi als Kühlflüssigkeit des Körpers. Die flächige Kompression der bekannten Kompressionskleidung wirkt diesbezüglich negativ, weil auf Grund der Kompression die Kapillare zusammengedrückt werden, wodurch der Bluttransfer reduziert ist. In Folge dessen führt die flächige Kompression zu einer Leistungsverringerung auf Grund nicht ausreichender Kühlung. Andere Kompressionsstrümpfe sind zum Beispiel JP2006225800, JP2007063722, WO2010/025186 (A54(3)), FR2852509, EP1475062, WO2005094738. Hier will die Erfindung Abhilfe schaffen. Der Erfindung liegt die Aufgabe zu Grunde, eine Kompressionskleidung zu schaffen, die zwar die Vorteile der bekannten Kompressionskleidung nutzt, gleichzeitig jedoch die Blutversorgung in einer Weise aufrecht erhält, die eine ausreichende Durchblutung der beanspruchten Muskeln ermöglicht. Gemäß der Erfindung wird diese Aufgabe dadurch gelöst, dass bereichsweise Mittel zur Kompression vorgesehen sind.

Mit der Erfindung ist eine Kompressionskleidung geschaffen, die zwar einen Druck auf die Haut ausübt, gleichzeitig jedoch die Blutversorgung der Muskeln lediglich in einer Weise beschränkt, die eine hohe Leistungsfähigkeit ermöglicht. Dies ist durch die lediglich bereichsweise erfolgende Kompression hervorgerufen. In den zu den Kompressionsmitteln benachbarten Bereichen findet keine Kompression statt, sodass dort eine unbeeinflusste Blutzirkulation möglich ist. Auf diese Weise ist eine Versorgung der beanspruchten Muskeln mit "gekühltem" Blut, das von dem inneren Organismus transportiert wird, ermöglicht. Trotz der hervorgerufenen Kompression bleibt die Kühlleistung des Körpers erhalten. Hierdurch ist die Leistungsfähigkeit der Sportler wesentlich verbessert.
Weise ist eine Versorgung der beanspruchten Muskeln mit "gekühltem" Blut, das von dem inneren Organismus transportiert wird, ermöglicht. Trotz der hervorgerufenen Kompression bleibt die Kühlleistung des Körpers erhalten. Hierdurch ist die Leistungsfähigkeit der Sportler wesentlich verbessert.

Die Stege bieten in einfacher Weise die Möglichkeit, eine bereichsweise Kompression hervorzurufen. Zudem können sie beliebig an dem Bekleidungsstück angeordnet werden.

In Weiterbildung der Erfindung sind die Stege durch eine Erhöhung der Materialstärke hervorgerufen. Hierdurch ist auf einfache Weise eine Herstellung der Stege ermöglicht. Bei Verwendung eines Gewebes als Material kann die Erhöhung der Materialstärke durch die Verwendung von Fäden größeren Durchmessers erfolgen oder auch durch eine Anhäufung von Fäden. Die Stege haben eine angenähert dreieckige Form. Hierdurch ist gewährleistet, dass die Stege lediglich mit einem sehr schmalen Abschnitt auf der Haut aufliegen. Dies verbessert die lediglich bereichsweise Kompression durch das Kleidungsstück. Die Stege sind in regelmäßigen Abständen angeordnet. Hierdurch ist eine symmetrische Ausgestaltung des Kleidungsstücks hervorgerufen, was einerseits die Wirkung des Kompressionskleidungsstücks erhöht, andererseits die Herstellung vereinfacht.

Die Stege der Kompressionskleidung können zudem mit einer Beschichtung versehen sein. Hier bietet sich die Verwendung verschiedener Materialien für die Beschichtung an, um unterschiedliche Wirkungen zu erzielen. So können beispielsweise durch Auswahl der Beschichtung fungizide oder antibakterielle Wirkungen erzielt werden; es ist ebenfalls möglich, durch gezielte Auswahl einer Beschichtung reibungsmindernde Effekte zu erzielen.

Eine weitere Verbesserung der Versorgung der beanspruchten Muskeln mit "gekühltem" Blut ist dadurch möglich, dass die Mittel zur Kompression Unterbrechungen aufweisen. Hierdurch ist einerseits eine unbeeinflusste Blutzirkulation in den zu den Kompressionsmitteln benachbarten Bereichen möglich, in denen keine Kompression stattfindet, andererseits in den Unterbrechungen, in denen ebenfalls keine Kompression stattfindet. Die Leistungsfähigkeit der Sportler ist dadurch weiter verbessert.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben.

Es zeigen:

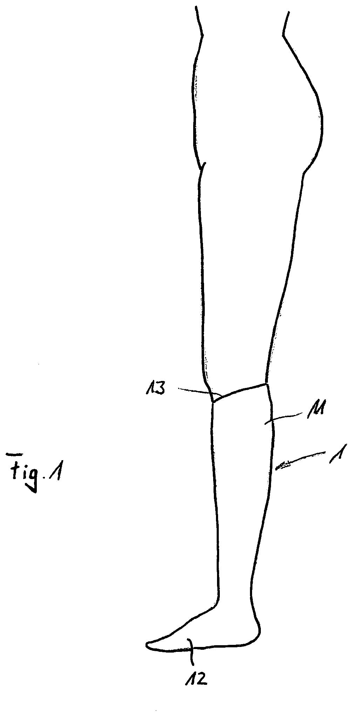

- Fig. 1: die Seitenansicht einer Kompressionskleidung in Form eines Kniestrumpfes, wie sie an einem menschlichen Körper angeordnet ist;

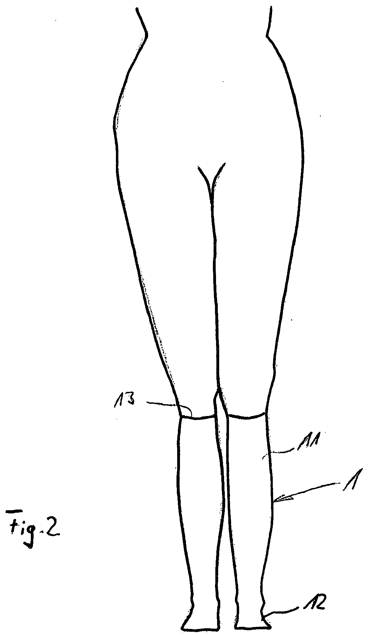

- Fig. 2: die Ansicht von vorne der in Fig. 1 dargestellten Kompressionskleidung;
- Fig. 3: die abschnittsweise Darstellung eines Schnitts durch die in Figur 4 dargestellte Kompressionskleidung entlang der Linie III-III;
- Fig. 4: die prinzipielle Darstellung der Anordnung von Stegen in vertikaler Ausrichtung; Diese Figur gehört nicht zur vorliegenden Erfindung
- Fig. 5: die prinzipielle Darstellung der Anordnung von Stegen in vertikaler Ausrichtung mit Unterbrechungen;
- Fig. 6: die prinzipielle Darstellung der Anordnung von Stegen in vertikaler Ausrichtung mit Unterbrechungen in anderer Ausbildung;
- Fig. 7: die prinzipielle Darstellung der Anordnung von Stegen in horizontaler Ausrichtung; Diese Figur gehört nicht zur vorliegenden Erfindung
- Fig. 8: die prinzipielle Darstellung der Anordnung von Stegen in horizontaler Ausrichtung mit Unterbrechungen;
- Fig. 9: die prinzipielle Darstellung der Anordnung von Stegen in horizontaler Ausrichtung mit Unterbrechungen in anderer Ausbildung;
- Fig. 10: die prinzipielle Darstellung der Anordnung von Stegen mit spiralförmiger Ausrichtung; Diese Figur gehört nicht zur vorliegenden Erfindung
- Fig. 11: die prinzipielle Darstellung der Anordnung von Stegen mit spiralförmiger Ausrichtung mit Unterbrechungen;
- Fig. 12: die prinzipielle Darstellung der Anordnung von Gewebestreifen in horizontaler Ausrichtung; Diese Figur gehört nicht zur vorliegenden Erfindung
- Fig. 13: die prinzipielle Darstellung der Anordnung von Gewebestreifen in horizontaler Ausrichtung mit Unterbrechungen;
- Fig. 14: die schematische abschnittsweise Darstellung der bereichsweisen Kompressionswirkung einer Kompressionskleidung nach der vorliegenden Erfindung und
- Fig. 15: die schematische abschnittsweise Darstellung der flächigen Kompressionswirkung einer Kompressionskleidung nach dem Stand der Technik.

Das gewählte Kleidungsstück 1 einer Kompressionskleidung ist in Form eines Kniestrumpfes ausgebildet, der an dem Unterschenkel eines menschlichen Beins angeordnet ist. Der Kniestrumpf weist einen Schaft 11 und ein Fußteil 12 auf. An dem dem Fußteil 12 abgewandten Ende ist ein Bund 13 vorgesehen.

An dem Kompressionskleidungsstück 1 sind bereichsweise Mittel zur Kompression vorgesehen. In den zu den Kompressionsmitteln benachbarten Bereichen findet keine Kompression statt. In diesen Bereichen ist eine unbeeinflusste Blutzirkulation möglich.

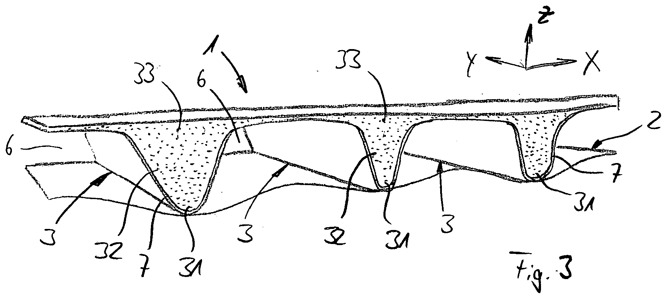

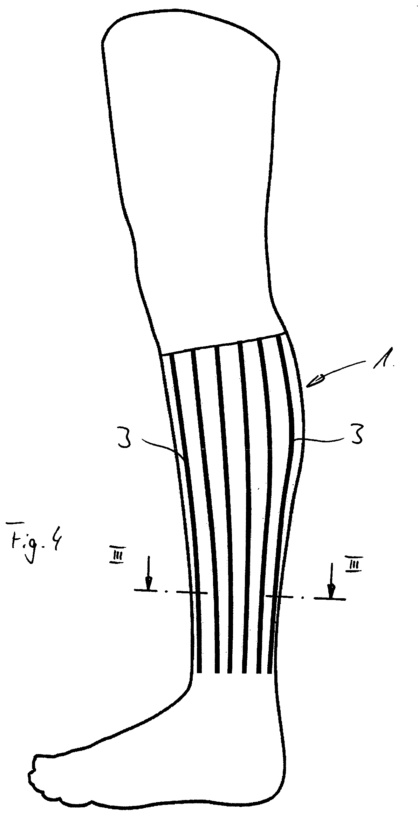

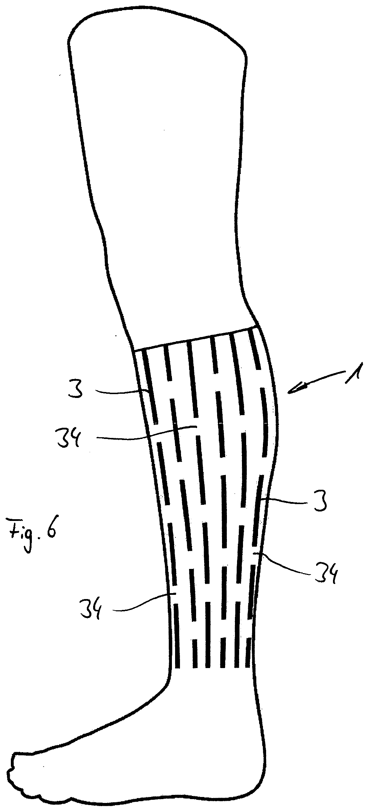

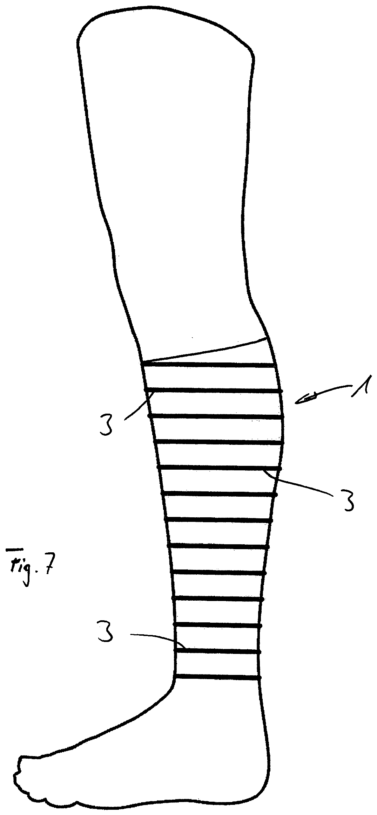

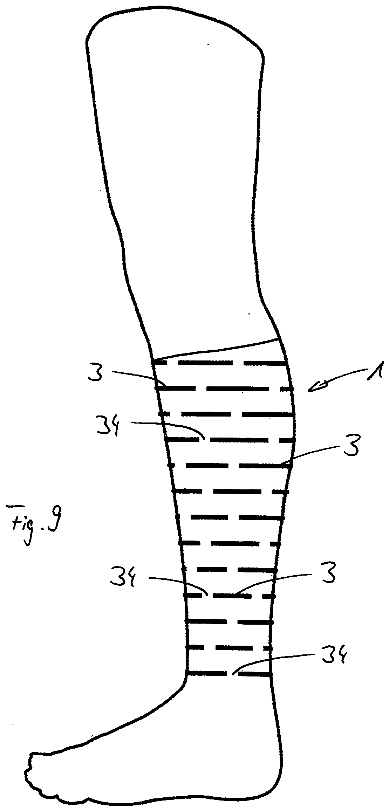

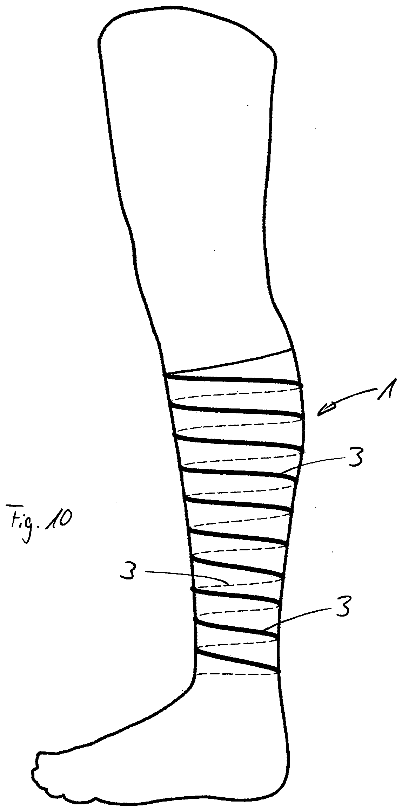

Bei dem Kompressionskleidungsstück 1 nach den Figuren 3 bis 11 (Figuren 4,7,10,12 gehören nicht zur vorliegenden Erfindung) sind die Mittel zur Kompression von Stegen 3 gebildet, die auf der der Haut 2 zugewandten Seite vorgesehen sind. Die Stege 3 sind in regelmäßigen Abständen angeordnet. Wie Figur 3 zu entnehmen ist, sind die Stege 3 durch eine Erhöhung der Materialstärke des Kleidungsstücks 1 hervorgerufen. Soweit es sich bei dem Material um ein Gewebe handelt, kann die Erhöhung der Materialstärke durch Verwendung von Fäden größeren Durchmessers zur Erstellung des Gewebes im Bereich der Stege hervorgerufen sein; es besteht auch die Möglichkeit, im Bereich der Stege 3 eine größere Anzahl von Fäden zu verwenden, woraus sich die erhöhte Materialstärke ergibt.

Die Stege 3 haben im Querschnitt eine angenähert dreieckige Form, sodass eine Spitze 31 hervorgerufen ist, die mit der Haut 2 in Kontakt steht. An die Spitze 31 schließen sich Schenkel 32 an, die sich bis zur Basis 33 des Steges 3 erstrecken. Die Basis 33 bildet im Ausführungsbeispiel die größte Breite der Stege 3 aus. Die Basis 33 bildet zudem gleichzeitig den Übergang in das restliche Material des als Kniestrumpf ausgebildeten Kleidungsstücks 1.

Das Material des Kleidungsstücks 1 ist aus für Kompressionskleidung üblicherweise verwendeten elastisch nachgebenden Materialien hergestellt. Das Material ist in sämtlichen Richtungen X, Y und Z elastisch ausgebildet. Das Material übt unter anderem eine Kompressionswirkung in Richtung der Haut 2 aus. Durch die die Stege 3 ausbildenden Materialverstärkungen ist durch die in Richtung der Haut 2 ausgeübte Kompressionskraft des Materials ein Druck der Spitzen 31 auf die Haut 2 bewirkt, wie dies in Figur 3 durch die wellenartige Form der Haut 2 angedeutet ist.

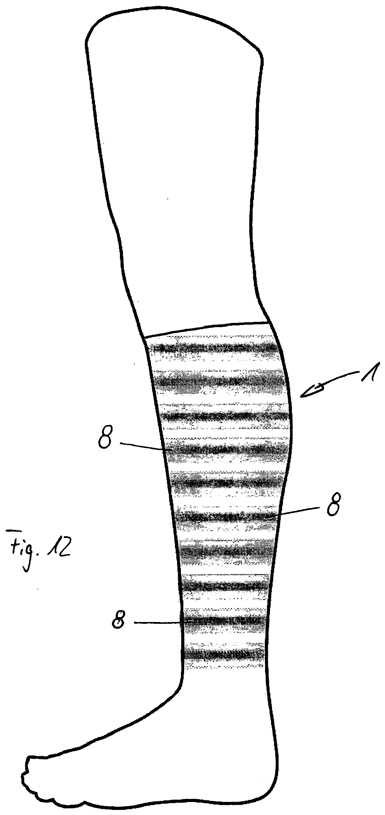

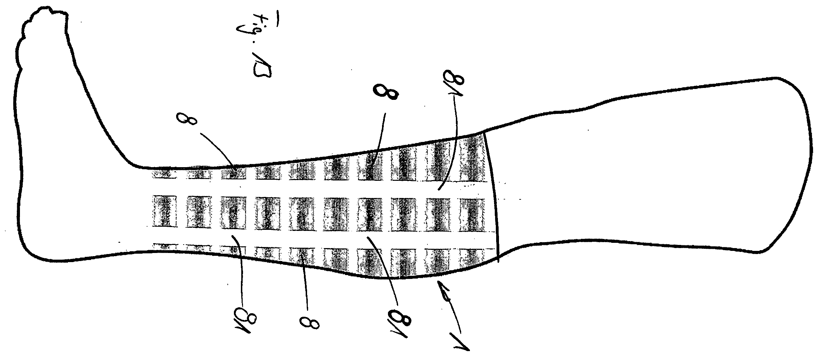

In den Figuren 12 und 13 (Die Figur 12 gehört nicht zur vorliegenden Erfindung) sind die Mittel zur Kompression von Gewebestreifen 8 gebildet, die eine zu dem Grundgewebe des Kleidungsstücks unterschiedliche Elastizität aufweisen. Dadurch sind abwechselnd weiche Gewebestreifen und feste Gewebestreifen vorgesehen, die eine unterschiedliche Kompressionswirkung haben. Das zwischen den benachbarten Gewebestreifen 8 verwendete Grundgewebe des Kleidungsstücks hat keine Kompressionswirkung. Durch die abwechselnde Verwendung von Gewebestreifen mit bzw. ohne Kompressionswirkung ist eine bereichsweise Kompression hervorgerufen.

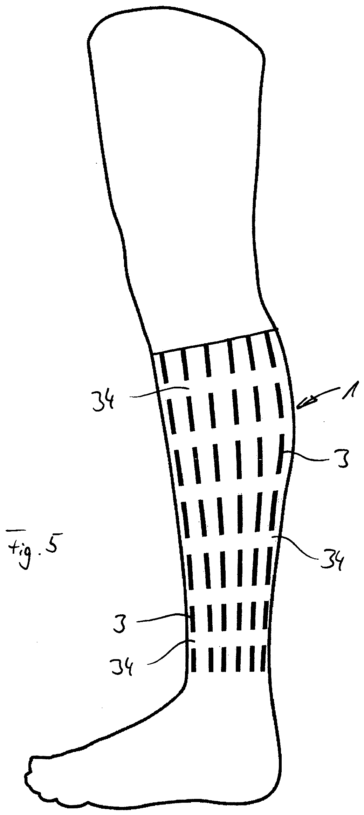

Die Mittel zur Kompression 3, 8 weisen Unterbrechungen 34, 81 auf (vgl. Figuren 5, 6, 8, 9, 11 und 13). Dadurch ist eine weitere Verbesserung der Versorgung der beanspruchten Muskeln mit "gekühltem" Blut möglich. In den Unterbrechungen 34, 81 kann nämlich eine unbeeinflusste Blutzirkulation erfolgen, wie dies auch in den zu den Kompressionsmitteln 3, 8 benachbarten Bereichen möglich ist.

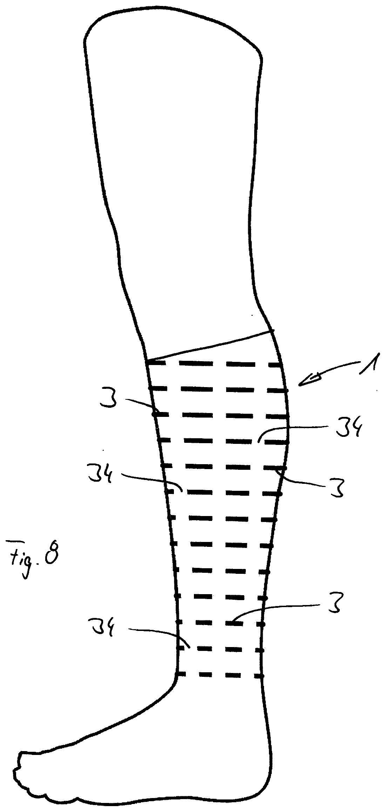

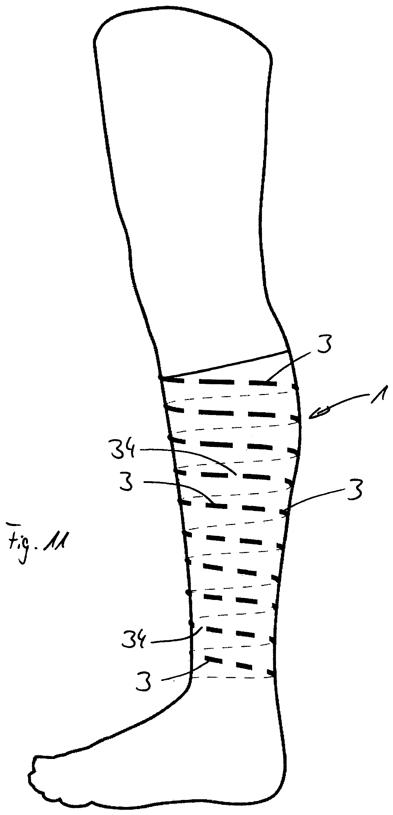

Die Ausrichtung der Mittel zur Kompression 3, 8 kann nahezu beliebig erfolgen. Beispielhaft sind in den Figuren 4 bis 13 (Die Figuren 4,7,10,12 gehören nicht zur vorliegenden Erfindung) verschiedene Ausrichtungen dargestellt. Neben der einfachen vertikalen Ausrichtung nach den Figuren 4 bis 6 besteht auch die Möglichkeit einer horizontalen Ausrichtung (vgl. Figuren 8 bis 9 und 13). Auch eine spiralförmige Ausrichtung der Mittel zur Kompression 3, 8 ist möglich, wie sie in der Figur 11 dargestellt ist. Allen Ausrichtungen ist gemeinsam, dass die Bereiche mil Kompressionswirkung im Wechsel zu Bereichen ohne Kompression angeordnet sind. Dadurch ist die erfindungsgemäße bereichsweise Kompression hervorgerufen. Wie den Figuren zu entnehmen ist, können unabhängig von der Ausrichtung der Mittel 3, 8 die Unterbrechungen 34, 81 vorgesehen sein, die die erfindungsgemäßen Wirkungen zusätzlich verstärken.

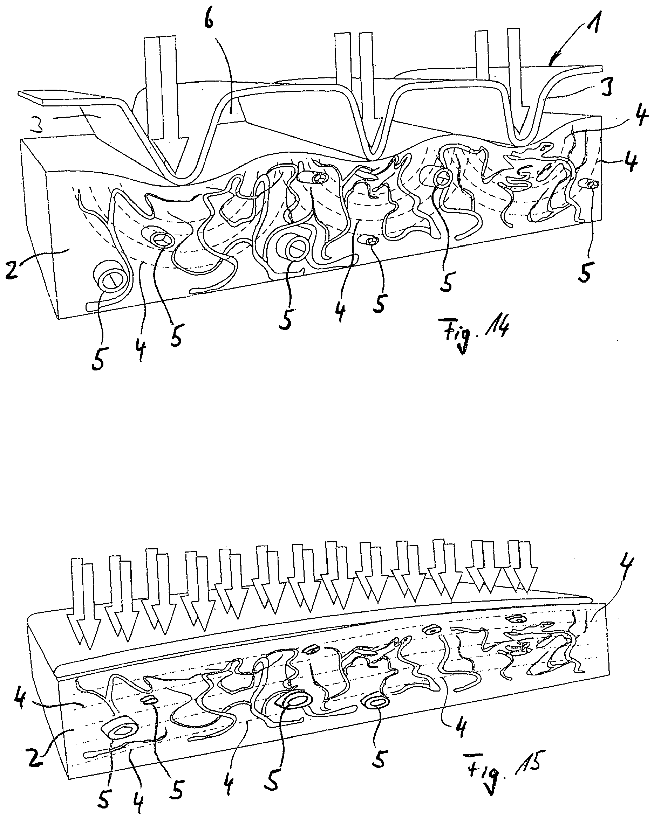

In den Figuren 14 und 15 ist schematisch die bereichsweise Kompressionswirkung nach der Erfindung der flächigen Kompressionswirkung nach dem Stand der Technik gegenübergestellt. Dabei ist in der Haut 2 der Kompressionsverlauf an Hand der Linien 4 angedeutet. Erkennbar findet eine Kompression der Haut 2 lediglich im Bereich der der Stege 3 bzw. der Gewebestreifen 8 statt. An diesen Stellen bleibt die Haut hell, wogegen im Bereich zwischen den den Stegen 3 bzw. den Gewebestreifen 8 die Haut 2 eine Rötung annimmt. Die Rötung der Haut macht die bessere Durchblutung im Bereich zwischen den Stegen 3 bzw. den Gewebestreifen 8 deutlich. Dies ist durch die sich erweiternden Kapillaren 5 bei körperlicher Anstrengung bewirkt.

Da die Stege 3 bzw. die Gewebestreifen 8 lediglich streifenförmig auf der Haut 2 aufliegen, werden die unter der Haut 2 befindlichen Kapillaren 5 kaum komprimiert, wie dies Figur 14 zu entnehmen ist. Dagegen ist bei den aus dem Stand der Technik bekannten Kleidungsstücken mit flächiger Kompression ein Zusammendrücken der Kapillare 5 hervorgerufen (vgl. Fig. 15), was eine Verschlechterung der Durchblutung zur Folge hat. Der Darstellung der Kompression nach dem Stand der Technik gemäß Figur 15 ist zu entnehmen, dass die Wirkung der Kompression, welche durch die Linien 4 verdeutlicht ist, gleichmäßig ohne Rücksicht auf die Lage der Kapillare 5 verläuft. Infolgedessen sind die Kapillare 5 zusammengedrückt, was sich aus dem flachen, ovalen Querschnitt der Kapillare 5 ergibt. Infolgedessen kann unter der Kompressionskleidung nach dem Stand der Technik die für eine Kühlung des Organismus erforderliche Blutzirkulation nicht aufrechterhalten werden, was sich negativ auf die Leistungsfähigkeit der Person auswirkt.

Die auf Grund der nicht komprimierten Kapillare 5 zwischen den Stegen 3 bzw. den Gewebestreifen 8 aufrechterhaltene Möglichkeit einer Blutzirkulation unter der Haut ermöglicht einen Temperaturaustausch, wodurch eine Kühlung des Organismus hervorgerufen ist. Durch die im Bereich zwischen benachbarten Stegen 3, der Haut 2 sowie dem Material des Bekleidungsstücks 1 hervorgerufenen und mit "6" gekennzeichneten tunnelartigen Ausbildungen besteht zudem die Möglichkeit, die auf der Haut 2 entstehende Wärme abzutransportieren. Darüber hinaus verhindert der Abstand zwischen dem Kleidungsstück 1 und der Haut 2 im Bereich zwischen den Stegen 3 ein Durchschwitzen des Kleidungsstücks. Vielmehr besteht lediglich im Bereich der Spitzen 31 der Stege 3 Kontakt zwischen dem Kleidungsstück 1 und der Haut 2, sodass das Kleidungsstück nur in diesem Bereich feucht werden kann. Auf diese Weise ist der Tragekomfort des erfindungsgemäßen Kleidungsstücks zusätzlich erhöht.

Der Tragekomfort ist außerdem dadurch erhöht, dass die Stege 3 mit einer Beschichtung 7 versehen sind. Bei der Beschichtung 7 kann es sich um verschiedene Arten handeln. So können unter anderem Funktionsbeschichtungen zum Einsatz kommen, die fungizide oder antibakterielle Wirkungen erzielen, beispielsweise Beschichtungen mit hohem Gold- oder Silberanteil. Es können aber auch andere Beschichtungen Anwendung finden, beispielsweise aus Polytetrafluorethylen. Dieser Werkstoff zeichnet sich unter anderem durch gute Gleiteigenschaften aus. Dies bewirkt bei der Verwendung als Beschichtung für die Stege 3, dass die Reibung der Kleidung 1 auf der Haut 2 deutlich reduziert ist, was ebenfalls zu einer Erhöhung des Tragekomforts beiträgt.

## Patentansprüche

1. Kompressionskleidung in Form eines Kniestrumpfes (1) mit einem Schaft (11) zum Tragen auf der Haut (2), wobei der Kniestrumpf (1) aus elastisch nachgebendem Material, besteht
wobei bereichsweise Mittel zur Kompression (3, 8) in verschiedenen Abschnitten des Kniestrumpfes (1) in regelmässigen Abständen, auf der der Haut (2) zugewandten Seite,vorgesehen sind,
**dadurch gekennzeichnet, dass**
die Mittel zur Kompression (3, 8)
entweder von Stegen (3) gebildet sind, die durch eine Erhöhung der Materialstärke im Bereich der Stege (3) hervorgerufen sind, wobei Fäden mit grösseren Durchmessern oder eine grössere Anzahl von Fäden zur Erstellung der Stege (3) gewählt sind,
wobei die Stege (3) im Querschnitt eine angenähert dreieckige Form haben und eine Spitze (31) aufweisen, an welche sich zwei Schenkel (32) bis zu einer Basis (33) anschliessen
oder
von Gewebestreifen (8) gebildet sind, die eine vom elastisch nachgebendem Material des Kniestrumpfes (1) unterschiedliche Elastizität aufweisen,
wobei die Mittel zur Kompression (3, 8) den Schaft (11) zwischen einem Fussteil (12) und einem Bund (13) umlaufend angeordnet sind
und Unterbrechungen (34, 81) entlang der Kompressionsmittel (3, 8) aufweisen derart, dass die Kompressionsmittel (3, 8) punktförmig bzw. streifenförmig auf der Haut (2) aufliegen, sodass die Mittel zur Kompression (3, 8) in ihrem Verlauf unterbrochen und von benachbarten Bereichen ohne Kompressionswirkung umgeben sind, in welchen die Blutzirkulation unbeeinflusst bleibt.

2. Kompressionskleidung nach Anspruch 1, **dadurch gekennzeichnet, dass** die Kompressionsmittel (3, 8) den Schaft (11) horizontal umlaufend angeordnet sind.

3. Kompressionskleidung nach Anspruch 1, **dadurch gekennzeichnet, dass** die Kompressionsmittel (3, 8) den Schaft (11) spiralförmig umlaufend angeordnet sind.

4. Kompressionskleidung nach einem der vorhergehenden Ansprüche, **dadurch gekennzeichnet, dass** die Stege (3) mit einer Beschichtung (7) versehen sind [vormals Anspruch 6], wobei die Beschichtung einen Gold-, Silber- oder Polytetrafluorethylen-Anteil aufweist.

## Claims

1. A compression garment in the form of a knee-stocking (1) with a leg section (11) to be worn on the skin (2), wherein the knee-stocking (1) is made of elastically yielding material,
wherein means for compression (3, 8) are provided locally at regular intervals in various sections of the knee-stocking (1) on the side facing the skin (2)
**characterized in that**
the means for compression (3, 8)
are formed either by stiffening elements (3) created by an increase in the material thickness in the area of the stiffening elements (3), wherein threads with larger diameters or a larger number of threads are selected to produce the stiffening elements (3),
wherein the stiffening elements (3) have an approximately triangular cross section and have an apex (31) from which two legs (32) adjoin a base (33),
or
are formed by fabric strips (8) which have an elasticity which differs from that of the elastically yielding material of the knee-stocking (1),
wherein the means for compression (3, 8) are arranged to encircle the leg section (11) between a foot part (12) and an edging section (13)
and have interruptions (34, 81) along the compression means (3, 8) in such manner that the compression means (3, 8) lie against the skin (2) in the manner of single points and/or strips, so that the course of the means for compression (3, 8) is discontinuous and they are surrounded adjacent areas without a compression effect, in which the circulation of the blood is unaffected.

2. The compression garment according to claim 1, **characterized in that** the compression means (3, 8) are arranged to extend around the leg section (11) horizontally.

3. The compression garment according to claim 1, **characterized in that** the compression means (3, 8) are arranged to extend around the leg section (11) spirally.

4. The compression garment according to any one of the preceding claims **characterized in that** the stiffening elements (3) are furnished with a coating (7) [previously Claim 6], wherein the coating has a content of gold, silver or polytetrafluoroethylene.

## Revendications

1. Vêtement de compression sous la forme d'un mi-bas (1) avec une tige (11) pour porter sur la peau (2), le mi-bas (1) étant composé d'un matériau élastiquement extensible,
des moyens étant prévus par zones pour compression (3, 8) sur le côté tourné vers la peau, à des intervalles réguliers dans différentes sections du mi-bas (1),
**caractérisé en ce que**
les moyens de compression (3, 8),
soit, sont formés par des nervures (3), qui sont engendrées par une augmentation de l'épaisseur du matériau dans la zone des nervures (3), des fils avec des diamètres plus grands ou un nombre plus important de fils pour l'élaboration des nervures (3) étant choisis,
les nervures (3) ayant dans la section transversale une forme à peu près triangulaire et comportant une pointe (31) à laquelle se raccordent deux branches (32) jusqu'à une base (33),
soit,
sont formées par des bandes de tissu (8) qui comportent une élasticité différente du matériau élastiquement extensible du mi-bas (1),
les moyens de compression (3, 8) étant disposés entourant périphériquement la tige (11) entre une partie du pied (12) et une union (13), et
comportant des interruptions (34, 81) le long des moyens de compression (3, 8) de telle sorte que les moyens de compression (3, 8) reposent sur la peau (2) sous forme de points ou de bandes de telle manière que les moyens de compression (3, 8) sont interrompus dans leur développement et sont entourés par des zones adjacentes sans effet compressif dans lesquelles la circulation sanguine reste sans être influencée.

2. Vêtement de compression selon la revendication 1, **caractérisé en ce que** les moyens de compression (3, 8) sont disposés entourant la tige (11) horizontalement.

3. Vêtement de compression selon la revendication 1, **caractérisé en ce que** les moyens de compression (3, 8) sont disposés entourant périphériquement la tige (11) en forme de spirale.

4. Vêtement de compression selon l'une quelconque des revendications précédentes, **caractérisé en ce que** les nervures (3) sont dotées d'un revêtement (7) [anciennement Revendication 6], le revêtement comportant une partie en or, en argent ou en polytétrafluoréthylène.
